(19) **Europäisches Patentamt / European Patent Office / Office européen des brevets**

(11) **EP 1 813 622 B1**

(12) **EUROPEAN PATENT SPECIFICATION**

(45) Date of publication and mention
of the grant of the patent:
**22.04.2015 Bulletin 2015/17**

(51) Int Cl.:
***C07H 13/06*** (2006.01)

(21) Application number: **05806883.4**

(86) International application number:
**PCT/JP2005/021269**

(22) Date of filing: **18.11.2005**

(87) International publication number:
**WO 2006/054708 (26.05.2006 Gazette 2006/21)**

(54) **SUCROSE FATTY ACID ESTER WITH LOW DEGREE OF SUBSTITUTION AND PROCESS FOR PRODUCING THE SAME**

SACCHAROSEFETTSÄUREESTER MIT NIEDRIGEM SUBSTITUTIONSGRAD UND VERFAHREN ZU DESSEN HERSTELLUNG

ESTER D'ACIDE GRAS DE SUCROSE PRÉSENTANT UN FAIBLE DEGRÉ DE SUBSTITUTION ET PROCÉDÉ POUR PRODUIRE CET ESTER

(84) Designated Contracting States:
**DE**

(30) Priority: **19.11.2004 JP 2004336310**

(43) Date of publication of application:
**01.08.2007 Bulletin 2007/31**

(73) Proprietor: **Mitsubishi Chemical Corporation
Chiyoda-ku
Tokyo 100-8251 (JP)**

(72) Inventors:
• **NAGAI, Yuki
Yokkaichi-shi,
Mie 5108530 (JP)**
• **KUZUI, Hiroshi
Yokkaichi-shi,
Mie 5108530 (JP)**

• **KIYOKAWA, Atsuo
Yokkaichi-shi,
Mie 5108530 (JP)**

(74) Representative: **Vossius & Partner
Patentanwälte Rechtsanwälte mbB
Siebertstrasse 3
81675 München (DE)**

(56) References cited:
EP-A1- 0 560 081    EP-A2- 0 346 845
GB-A- 2 256 869    JP-A- 5 178 878
JP-A- 5 222 084    JP-A- 6 157 575
JP-A- 7 118 285    JP-A- 7 228 590
JP-A- 10 017 588    US-A- 4 611 055

EP 1 813 622 B1

**Description**

TECHNICAL FIELD

The present invention relates to a process for producing a sucrose fatty acid ester having a low degree of substitution. More particularly, the invention relates to a process for producing a low-substitution-degree sucrose fatty acid ester having a high hydrophilicity and which is useful in the field of foods, etc.

BACKGROUND ART

A sucrose fatty acid ester (hereinafter sometimes abbreviated to SE) is a substance in which at least part of the eight hydroxyl groups of a sucrose molecule framework has been esterified with a fatty acid. Since the degree of esterification of an SE has a distribution, it is usually expressed in terms of average degree of substitution (hereinafter also referred to simply as "degree of substitution").

SEs have a wide range of values of HLB, which is a measure of hydrophilicity/hydrophobicity, and are in extensive use as surfactants for foods. SEs having low degrees of substitution have a high HLB value, i.e., high hydrophilicity, and are used as, e.g., emulsion stabilizers for the oil-in-water type (O/W type) in, e.g., milked coffee. These SEs are highly valuable in the market for emulsifying agents for foods and there is a large demand for these.

Many proposals have been made on processes for producing an SE. These processes are roughly divided into the following three kinds.

A first process is a process called a solvent process. This is a process in which a reaction is conducted using a good solvent for both of sucrose and a fatty acid derivative, such as, e.g., dimethylformamide (DMF) or dimethyl sulfoxide (DMSO). For example, sucrose is reacted with the methyl ester of a fatty acid in DMSO at about 90°C in the presence of an alkali catalyst. Although methanol generates as a by-product in this reaction, the reaction is conducted while removing the methanol. Especially when the SE is to be used in a food application, it is necessary to diminish the residual solvent. Consequently, when an SE having a low degree of substitution is to be produced by this process, a solvent removal apparatus employing multistage extraction or crystallization is necessary and a complicated operation technique is required. Furthermore, even when a solvent removal treatment is performed, the solvent actually remains in a slight amount (about 0.3 ppm in the case of, e.g., DMSO).

A second process is a process generally called a microemulsion process. This is a process in which a solution prepared by dissolving sucrose in propylene glycol, water, or the like and a fatty acid derivative, e.g., the methyl ester of a fatty acid, are converted to an exceedingly fine dispersion system, i.e., a microemulsion, with an emulsifying agent such as a fatty acid soap and this dispersion system is reacted after solvent removal (see patent document 1). However, the step of removing the solvent while maintaining the microemulsion state requires a high level of technique. In addition, although the reaction is conducted generally at a temperature as high as 100-150°C, sucrose is apt to decompose upon heating to 100°C or higher, especially 120°C or higher.

The microemulsion process is not in use for the production of an SE having a low degree of substitution. The reasons for this are presumed to be as follows. For obtaining an SE having a low degree of substitution, it is necessary to use sucrose in a large excess relative to the amount of the fatty acid derivative to be esterified. Furthermore, since the amount of the fatty acid methyl ester decreases with the progress of the reaction, the liquid reaction mixture increases in viscosity and becomes difficult to stir when the reaction is to be carried out almost completely. In this case, a measure should be taken, such as elevating the temperature or using a special stirrer. Moreover, in the case where propylene glycol is used as a solvent, the propylene glycol remains disadvantageously in the liquid reaction mixture.

A third process is a process in which sucrose is directly mixed with a fatty acid ester without using a solvent and the reactants are reacted by heating the mixture to 100-150°C. This process is called a direct process. Sucrose does not have an affinity for fatty acid esters. Because of this, how to mix the two raw materials and bring them into the state of being capable of smoothly reacting is important in the direct process. In general, this reaction is conducted using an alkali soap as a mixing aid while heating the mixture to a temperature which is equal to or higher than that in the microemulsion process. Consequently, sucrose decomposition by heating is apt to occur. The direct process is not in use for the production of an SE having a low degree of substitution. The reasons why the direct process is not in use for the production of an SE having a low degree of substitution are thought to be the same as in the case of the microemulsion process. A technique of the direct process has also been proposed in which an SE and sucrose are subjected to transesterification (see patent documents 2 and 3). However, this technique also has the problem concerning viscosity increase described above.

Patent Document 4 discloses a process for producing a high-monoester sucrose higher fatty acid ester with a monoester content of not less than 90% which comprises dissolving a sucrose higher fatty acid ester in a solvent having a specific inductive capacity, at 25°C, of 30 to 50, the amount of said solvent being 1 to 12 parts by weight based on each part by weight of said sucrose higher fatty acid ester, at an elevated temperature, separating a supernatant after cooling, and removing the solvent from said supernatant.

Patent Document 1: JP-B-51-14485
Patent Document 2: JP-B-4-65080
Patent Document 3: JP-A-59-78200
Patent Document 4: EP-A-0 560 081

DISCLOSURE OF THE INVENTION

PROBLEMS THAT THE INVENTION IS TO SOLVE

An object of the invention is to provide processes which are useful for obtaining a sucrose fatty acid ester having high quality and a low degree of substitution and which is suitable for use as, e.g., an emulsifying agent for foods, and which processes eliminate the necessity of the complicated solvent removal operation in conventional processes and are industrially superior.

MEANS FOR SOLVING THE PROBLEMS

The present inventors made intensive investigations in order to eliminate the problems of the first to third process as described above. As a result, they have found that when a solvent having specific properties and an activated sucrose are used in the transesterification reaction of sucrose with an SE, then a low-substitution-degree SE of high quality can be produced industrially advantageously. The invention has been thus achieved.

A gist of the invention resides in a production process of a sucrose fatty acid ester having a low degree of substitution, which comprises lowering a degree of substitution of a raw sucrose fatty acid ester, wherein the production process comprises reacting the raw sucrose fatty acid ester with an activated sucrose in the presence of a solvent having a dielectric constant at 20°C of 35.0 or lower and having no hydroxyl group

whereby the average degree of substitution of the raw sucrose fatty acid ester is lowered by 0.2 or more, and wherein the sucrose fatty acid ester having a low degree of substitution to be yielded has an average degree of substitution of 1.0 to 2.0.

Another gist of the invention resides in a production process of a sucrose fatty acid ester having a low degree of substitution, which comprises lowering a degree of substitution of a raw sucrose fatty acid ester, wherein the production process comprises reacting the raw sucrose fatty acid ester with an activated sucrose in the presence of a solvent having a boiling point at ordinary pressure of 150°C or lower and a solubility in water at 20°C of 50 g/100 mL or lower and having no hydroxyl group

whereby the average degree of substitution of the raw sucrose fatty acid ester is lowered by 0.2 or more, and wherein the sucrose fatty acid ester having a low degree of substitution to be yielded has an average degree of substitution of 1.0 to 2.0.

By one of these processes, a sucrose fatty acid ester is obtained which has an average degree of substitution of 1.0 to 1. 6 and contains a solvent (solvent (a)) having a dielectric constant at 20°C of 35.0 or lower and having no hydroxyl group, and contains no solvent other than the solvent (a) (provided that water, MeOH, and EtOH are excluded). By the other of those processes, a sucrose fatty acid ester is obtained which has an average degree of substitution of 1.0 to 1.6 and contains a solvent (solvent (b)) having a boiling point at ordinary pressure of 150°C or lower and a solubility in water at 20°C of 50 g/100 mL or lower and having no hydroxyl group, and contains no solvent other than the solvent (b) (provided that water, MeOH, and EtOH are excluded). In both processes, the average degree of substitution of the raw sucrose fatty acid ester is lowered by 0.2 or more and the sucrose fatty acid ester having a low degree of substitution to be yielded has an average degree of substitution of 1.0 to 2.0.

Preferred embodiments of the processes of the invention include the following. The solvent has a dielectric constant at 20°C of 1.1 to 20.0.

The raw sucrose fatty acid ester has an average degree of substitution of 1.5 to 5.0. The raw sucrose fatty acid ester is a fatty acid having 8-22 carbon atoms. The raw sucrose fatty acid ester is an ester of palmitic acid. The reaction of the raw sucrose fatty acid ester with the activated sucrose is conducted in the presence of a fatty acid soap. The sucrose fatty acid ester having a low degree of substitution has an ash content of 6.0% by weight or lower. The sucrose fatty acid ester having a low degree of substitution is one in which 70 to 100% of the fatty acid(s) contained as a component of the ester is palmitic acid.

ADVANTAGES OF THE INVENTION

According to the processes of the invention, a sucrose fatty acid ester having a low degree of substitution can be obtained without using a solvent difficult to remove. Namely, the reaction solvent in a low-substitution-degree sucrose fatty acid ester can be removed to about 1 ppm by a simple operation, e.g., a distillation/evaporation operation. Consequently, the complicated operations necessary for obtaining a high-quality product in the solvent process heretofore in use, such as multistage solvent extraction and a crystallization operation, are unnecessary. As a result, the cost of equipment investment can be reduced to an exceedingly low level and a greatly simplified operation technique suffices. Especially when the reaction solvent is a poor solvent for sucrose, this reaction solvent as it is may be used as a solvent for removing the unreacted sucrose from the SE synthesis reaction mixture by phase separation, etc. This can reduce the number of solvents to be used for SE production, whereby solvent recovery can be simplified and the cost of equipment investment can be reduced.

As in the solvent process heretofore in use, the reaction in the processes of the invention can be conducted at a lower temperature than in the direct process and microemulsion process. Low reaction temperatures are less apt to cause the thermal decomposition of sucrose and are hence suitable for the production of a high-quality SE, in particular, an SE having a low degree of substitution. Consequently, the processes of the invention are exceedingly useful as industrial processes for producing a high-quality SE having a low degree of substitution.

BRIEF DESCRIPTION OF THE DRAWINGS

[Fig. 1] Fig. 1 is a diagrammatic view of the apparatus used for comparing the evaporation rates of MEK and DMSO.
[Fig. 2] Fig. 2 is a graphic presentation showing a comparison between the evaporation rates of MEK and DMSO.

BEST MODE FOR CARRYING OUT THE INVENTION

The invention will be explained below in detail. The following explanations on constituent elements are for typical embodiments of the invention and should not be construed as limiting the invention.

The processes of the invention are ones in which the transesterification reaction of an SE with sucrose is conducted using an activated sucrose as the sucrose and using a solvent having specific properties to thereby produce a sucrose fatty acid ester having a lower degree of substitution than the raw sucrose fatty acid ester.

Raw Sucrose Fatty Acid Ester (raw SE)

The degree of substitution of the sucrose fatty acid ester to be used as a starting material (raw SE) in the invention may be any value of 1-8 as long as the requirements in claim 1 are met.

A mixture of sucrose fatty acid esters differing in the degree of esterification may be used. For producing a sucrose fatty acid ester having a low degree of substitution (low-substitution-degree SE), the lower limit of the average degree of substitution of the raw SE is preferably 1.5, more preferably 2.0, and the upper limit thereof is preferably 5.0.

Processes for producing the raw SE are not particularly limited. A single SE obtained by, e.g., any of the processes described hereinabove may be used, or a mixture of SEs obtained by two or more processes may be used. For obtaining a high-quality sucrose fatty acid ester having a low degree of substitution by the processes of the invention, it is preferred to use as the raw SE an SE which contains no solvent difficult to remove and has been produced by the emulsion process, direct process, etc.

The fatty acid constituting the ester bonds of the SE is not particularly limited. A mixture of two or more fatty acids in any desired proportion may be used. Usually, use can be made of saturated or unsaturated fatty acids having 4-30 carbon atoms, such as lauric acid, myristic acid, palmitic acid, oleic acid, stearic acid, and erucic acid. From the standpoint of the function required of emulsifying agents, saturated or unsaturated fatty acids having 8-22 carbon atoms are preferred of those fatty acids. For use as an emulsifying agent for foods or the like, palmitic acid or stearic acid is preferred. Especially for use in emulsification for coffee beverages or the like, palmitic acid is suitable because it has a bacteriostatic action. It is desirable that palmitic acid accounts for preferably at least 70%, more preferably at least 80% of the fatty acid(s) constituting the ester bonds of the SE. The upper limit of the proportion thereof is 100%.

Reaction Solvent

The reaction solvent to be used in the processes of the invention usually has a dielectric constant at 20°C of 35.0 or lower. Dielectric constant is an index to molecular polarity, and is usually measured with an external electric field having a frequency of about 1 MHz. Specific values of dielectric constant are given in, e.g., Kagaku Binran, 5th revised edition II, pp. 619-623 and Kagaku Binran, 4th revised edition II, pp.498-501. The lower limit of the dielectric constant at 20°C of the reaction solvent is preferably 1.1, more preferably 2.3. The upper limit thereof is preferably 20.0, more preferably 19.0.

Furthermore, the reaction solvent to be used in the processes of the invention is one which has no hydroxyl group because this solvent does not form an ester during the reaction. The reaction solvent to be used may consist of a single compound or may be a mixture of two or more compounds.

The solubility in water of the reaction solvent to be used in the processes of the invention specifically is preferably 50 g/100 mL or lower, especially preferably 30 g/100 mL or lower, at 20°C. The lower limit of the solubility of the reaction solvent in water is usually 0.001 g/100 mL. In general, solvents having a dielectric constant at 20°C of 35.0 or lower have low polarity and hence low solubility in water, and solvents having low solubility in water are poor solvents for sucrose. In the processes of the invention, the unreacted sucrose remaining after completion of the reaction can be removed by phase separation with water, crystallization, etc. When the reaction solvent is low in solubility in water and in the solubility of sucrose therein, it is easy to remove unreacted sucrose together with water by conducting phase separation to dissolve the sucrose in the aqueous phase side. Furthermore, when the reaction solvent is a poor solvent for sucrose, it is also easy to remove sucrose by crystallization. Consequently, the solvent to be used in the processes of the invention preferably is a poor solvent for sucrose.

It has hitherto been thought that the solvent to be used for an SE synthesis reaction should be one in which sucrose dissolves. It has been thought that using a poor solvent for sucrose to react sucrose with a fatty acid derivative, e.g., the methyl ester of a fatty acid, or with an SE or the like is substantially impossible. In particular, the solvents heretofore in use for SE synthesis reactions have been thought to be practically unusable as reaction solvents for the synthesis of an SE having a low degree of substitution because of an exceedingly low reaction rate. In general, the solvents in common use in SE synthesis reactions, such as DMF, DMSO, and propylene glycol, can be mingled with water in any proportion at 20°C. In the processes of the invention, however, such poor solvents for sucrose are usable because an activated sucrose is used. Incidentally, the solvent in the processes of the invention is used mainly for the purpose of reducing the viscosity of the reaction mixture solution.

Furthermore, solvents having a dielectric constant at 20°C of 35.0 or lower usually have a low boiling point because of their low polarity. The reaction solvent to be used in the processes of the invention preferably has a boiling point which is not excessively lower than the reaction temperature. The processes of the invention are usually practiced at ordinary pressure while keeping the system at about 90°C. The boiling point of the solvent is therefore preferably 70-150°C, more preferably 70-120°C, at ordinary pressure. However, since the reaction temperature can be varied according to reaction conditions, e.g., reaction pressure, it is possible to use a solvent having a lower or higher boiling point than that. Incidentally, the boiling points at ordinary pressure of solvents in common use for SE production are as follows: 153.0°C for DMF, 189.0°C for DMSO, 187.4°C for propylene glycol, and 100.0°C for water.

Examples of the reaction solvent to be used in the processes of the invention include the following. However, the reaction solvent to be used in the processes of the invention should not be construed as being limited to these. The values in each parenthesis indicate dielectric constant at 20°C, solubility in water at 20°C (g/100 mL), and boiling point at ordinary pressure, respectively. Ketones such as methyl ethyl ketone (18.6, 29.0, 79.8) and acetone (21.0, (freely mingled with water), 56.1); hydrocarbons such as toluene (2.4, 0.045, 110.8), xylene (2.6, 0.02, 144.6), and hexane (1.9, 0.0013, 68.9), and chlorinated hydrocarbons such as chloroform (4.8, 0.8, 61.3) and chlorobenzene (5.7, 0.05, 131.9). In the case where the SE having a low degree of substitution is to be used as a food additive, methyl ethyl ketone or hydrocarbons are preferred of those because the reaction temperature can be set at 80-90°C and from the standpoints of chemical stability, etc. More preferred are methyl ethyl ketone and toluene. Especially preferred is methyl ethyl ketone.

Activated Sucrose

The sucrose to be reacted with an SE in the processes of the invention is an activated sucrose. The activated sucrose is a substance in which at least part of the protons of the hydroxyl groups of sucrose is bonded to an alkali metal or an alkaline earth metal.

Examples of the activated sucrose include (1) sucrate anions formed by replacing at least part of the protons in the hydroxyl parts of sucrose with an alkali metal and/or alkaline earth metal and (2) complexes comprising a combination of one or more metals (alkali metals and/or alkaline earth metals) and/or metal compounds (e.g., alkoxides, carbonates, hydrogen carbonates, hydrides, or hydroxides of an alkali metal and/or alkaline earth metal) with sucrose. Either one activated sucrose or a mixture of two or more activated sucroses may be used in the processes of the invention. The activated sucrose includes emulsifying agents such as soaps comprising those substances.

The activated sucrose is presumed to be in an amorphous state resulting from the cleavage of part of the hydrogen bonds of the sucrose. Consequently, a mere mixture of sucrose and an alkali metal or alkaline earth metal is not included in the activated sucrose.

The alkali metal and alkaline earth metal with which one or more protons have been replace in the activated sucrose preferably are alkali metals such as sodium and potassium, and especially preferably are potassium, from the standpoints of catalytic ability in transesterification and use for foods. These metals preferably are in the form of a hydroxide because the hydroxides are apt to dissolve in the water and alcohol to be used in preparing the activated sucrose.

That a sucrose has been activated can be ascertained by crystal structure analysis such as X-ray diffractometry, near infrared spectrophotometry, etc.

Various methods for sucrose activation have been proposed so far. The activated sucrose to be used in the processes of the invention may be a sucrose activated by any of these known methods. It may be one obtained by a method other than those.

Simple methods for preparing an activated sucrose include, for example, the following method.

Namely, it is a method which comprises mixing sucrose and a compound of an alkali metal or alkaline earth metal (e.g., a salt such as hydroxide) with water and an alcohol (e.g., methanol, ethanol, or the like) to sufficiently dissolve the solids and then drying/solidifying the solution with an evaporator or the like to thereby remove the water and alcohol to 0.1% by weight or lower. The proportion (molar ratio) of the compound of an alkali metal or alkaline earth metal to the sucrose in preparing an activated sucrose is preferably 5.0 or lower, more preferably 1.0 or lower, especially preferably 0.2 or lower, because such a proportion makes the sucrose less apt to be decomposed by heating. The lower limit thereof is usually 0.01.

The amount of the water to be used in this method is not particularly limited as long as the sucrose can be dissolved. In this method, the alcohol is used for the purpose of removing the water from the activated sucrose by azeotropy. Consequently, the amount of the alcohol to be used also is not particularly limited as long as that purpose is achieved. Furthermore, the sequence of mixing sucrose with those substances is not limited. It is, however, preferred that the drying/solidification and solvent removal be conducted at 90°C or lower, especially about 60°C, because sucrose decomposition is less apt to occur at such temperatures.

When, for example, a fatty acid soap is to be used as an emulsifying agent in producing a raw SE, then a fatty acid soap and a combination of a fatty acid, alkali metal (compound), fatty acid monoglyceride, etc. as a precursor for the soap may be incorporated beforehand also in the activation of sucrose.

Besides the method described above, examples of sucrose activation methods include the methods described in documents such as ARNI et al., J. Appl. Chem., 9, March, 1959, 186-192; and J.A. Rendleman, Jr., Adv. In Carbohydr. Chem., 21(1966), 209-269.

Substances other than Starting Materials and Solvent

In the processes of the invention, substances other than the starting materials and solvent may be present in the reaction system as long as these substances do not considerably inhibit the transesterification reaction of an activated sucrose with a raw SE. Specifically, an emulsifying agent such as, e.g., a fatty acid soap, fatty acid monoglyceride, or precursor for these may be supplied to the reaction system together with the activated sucrose. The kind and amount of the emulsifying agent are not limited as long as the emulsifying agent is effective in raw-material mixing. As the fatty acid soap is usually used a fatty acid potassium soap, fatty acid sodium soap, or the like. The fatty acid soap preferably is one formed from a fatty acid having 4-22 carbon atoms and an alkali metal because this soap is highly effective in emulsifying and mixing the starting materials and the solvent. The amount of the fatty acid soap to be used is preferably such that the lower limit thereof is 5% by weight, especially 10% by weight, and the upper limit thereof is 60% by weight, especially 50% by weight, based on the total amount of the raw SE, activated sucrose, and fatty acid soap because such amounts are highly effective in emulsifying and mixing the starting materials and the solvent. The fatty acid monoglyceride preferably is an ester of a fatty acid having 4-22 carbon atoms with glycerol because this glyceride is highly effective in emulsifying and mixing the starting materials and the solvent. The fatty acid monoglyceride may contain a diglyceride and a triglyceride as part thereof.

Use may be made of a method in which an activated sucrose and the reaction solvent according to the invention are added to a reaction mixture resulting from a reaction conducted by the direct process (the reaction mixture is in a state in which an SE, sucrose, fatty acid soap, catalyst, fatty acid methyl ester, etc. are present) to cause the SE in the reaction mixture to undergo a transesterification reaction and thereby obtain an SE having a low degree of substitution.

Production Processes

The processes of the invention are explained.

In the transesterification reaction according to the invention, the value (average) by which the degree of substitution of a raw SE is lowered to that of a low-substitution-degree SE is 0.2 or more, and desirably as follows. The lower limit thereof is preferably 0.5, more preferably 0.8. The upper limit of the value by which the degree of substitution is lowered is generally 4.0, preferably 1.2. The value by which the degree of substitution is lowered by the processes of the invention depends on the kind and degree of substitution of the raw SE, kind of the activated sucrose, amount of the activated sucrose used, kind of the solvent, reaction conditions such as reaction temperature and reaction time, degree of substitution of the target low-substitution-degree SE, etc.

The amount of the activated sucrose to be used in the transesterification reaction in the processes of the invention may be suitably regulated so that the degree of substitution of the SE is lowered to a desired value.

The amount of the solvent to be used in the reaction is regulated to a value which enables the liquid reaction mixture to be stirred even when the viscosity of the liquid reaction mixture has increased with the progress of the reaction. A too large solvent amount is undesirable because solvent removal after the reaction requires much time. Specifically, the amount of the solvent to be used is generally preferably 10-70% by weight, more preferably 40-70% by weight, based on the whole liquid reaction mixture.

The reaction temperature may be any temperature as long as the degree of substitution of the SE can be lowered by the transesterification reaction according to the invention. Higher reaction temperatures are preferred from the standpoint of reaction rate. From the standpoint of the quality of the low-substitution-degree SE to be obtained, lower reaction temperatures are preferred because sucrose decomposition is less apt to occur. From these standpoints, the reaction temperature in the case of ordinary pressure may be generally 80-110°C, preferably about 90°C. The reaction pressure may be any of ordinary pressure, a reduced pressure, and an elevated pressure as long as the esterification reaction according to the invention proceeds. It should, however, be noted that the reaction at an exceedingly elevated pressure not only necessitates an expensive apparatus but tends to result in sucrose decomposition. The reaction may be conducted in any atmosphere as long as the atmosphere does not considerably inhibit the reaction according to the invention. However, an inert atmosphere such as $N_2$ or argon is preferred from the standpoint of safety.

The reaction time is a time period in which the degree of substitution of the SE decreases to a desired value before completion of the transesterification reaction according to the invention. The reaction time may be suitably selected according to the value by which the degree of substitution is to be lowered, other reaction conditions, e.g., reaction time, etc. Specifically, the reaction time is generally 1 hour or longer, preferably 3 hours or longer, and is generally 15 hours or shorter, preferably 10 hours or shorter. The reason why the rate of the reaction for synthesizing a low-substitution-degree SE using an activated sucrose is not exceedingly low even when the reaction solvent is a poor solvent for sucrose is unknown. Usually, sucrose molecules have hydrogen bonds formed between hydroxyl groups thereof. It is presumed that an activation treatment of sucrose cleaves these hydrogen bonds and makes the hydroxyl groups of the sucrose molecules apt to come into contact with the ester parts of the SE and this prevents the reaction rate from becoming exceedingly low.

The liquid reaction mixture is usually stirred. A suitable method of stirring may be selected according to the viscosity of the liquid reaction mixture, etc. For example, when the solvent amount is about 70% by weight of the liquid reaction mixture, this reaction mixture is stirred with an anchor-type or semicircular stirring blade in many cases. When stirring is difficult, the solvent amount may be increased. The rate of stirring is not limited as long as the mixing of the reaction mixture is accelerated. Usually, the stirring rate is 10-1, 000 rpm. It is desirable to use a reactor in which the solvent which vaporizes during the reaction can be wholly refluxed to the reaction vessel.

An end point of the reaction can be ascertained based on, e.g., a viscosity change of the reaction mixture solution. After completion of the reaction, the reaction mixture is usually cooled rapidly or subjected to a post-treatment such as neutralization with an acid.

In the solvent process heretofore in use, a good solvent for sucrose and for fatty acid derivatives, such as DMF, is used and a special apparatus and a complicated unit operation were necessary for removing the solvent from the SE obtained by the reaction. In contrast, in the processes of the invention, a solvent having low polarity is used. Because this solvent usually has a relatively low boiling point, the solvent remaining in the SE after the reaction can be removed by a simple operation such as a distillation or evaporation operation. The amount of the residual solvent in the low-substitution-degree SE obtained by each process of the invention can be determined by gas chromatography. The amount of the residual solvent in the low-substitution-degree SE obtained by each process of the invention can be reduced to preferably about several parts per million, more preferably about 1 ppm.

The same solvent as the reaction solvent used in a process according to the invention can be used also in a post-treatment after the reaction in the process of the invention. Namely, a solvent usable as a reaction solvent in the process of the invention is further added to or otherwise contacted with the reaction mixture after completion of the reaction in the process of the invention and, according to need, water or the like is furthermore added to or otherwise contacted with the reaction mixture to thereby conduct extraction by phase separation, a crystallization operation, etc. Thus, the unreacted sucrose and the fatty acid soap can be removed from the reaction product and process simplification can be attained.

Sucrose Fatty Acid Ester Having Low Degree of Substitution (low-substitution-degree SE)

The degree of substitution of the low-substitution-degree sucrose fatty acid ester to be obtained by each process of the invention varies depending on the application thereof and is 1.0-2.0, preferably 1.0-1.6, more preferably 1.0-1.4.

According to each process of the invention, a sucrose fatty acid ester having a low degree of substitution can be obtained without using a solvent difficult to remove from the SE yielded. Namely, an SE synthesized by, e.g., the emulsion process or direct process without using a solvent difficult to remove is used as a raw material and subjected to the reaction for lowering the degree of substitution according to the invention, whereby a low-substitution-degree SE containing no solvent other than the solvent used in the process of the invention (provided that water, MeOH (methanol), and EtOH (ethanol) are excluded) can be obtained. There is a possibility that water might be contained in the low-substitution-degree SE of the invention because there are cases where the activated sucrose remaining unreacted is removed by water extraction in a post-treatment in the process of the invention. Furthermore, MeOH and EtOH may be contained in the low-substitution-degree SE of the invention because there is a possibility that they might generate as by-products through esterification reaction.

Specifically, by one process of the invention, a low-substitution-degree SE can be obtained which has an average degree of substitution of 1.0-1.6 and contains a solvent (solvent (a)) having a dielectric constant at 20°C of 35.0 or lower and having no hydroxyl group and which contains no solvent other than the solvent (a) (provided that water, MeOH, and EtOH are excluded). Furthermore, by the other process of the invention, an SE can be obtained which has an average degree of substitution of 1.0-1.6 and contains a solvent (solvent (b)) having a boiling point at ordinary pressure of 150°C or lower and a solubility in water at 20°C of 50 g/100 mL or lower and having no hydroxyl group and which contains no solvent other than the solvent (b) (provided that water, MeOH, and EtOH are excluded). That no solvent is contained can be ascertained by gas chromatography as a simple and convenient method. When higher accuracy is desired, that no solvent is contained can be ascertained by mass spectrometry.

In the processes of the invention, the reaction can be caused to proceed while the sucrose is being inhibited from decomposing. In case where the sucrose is decomposed, the low-substitution-degree SE obtained disadvantageously contains monosaccharides, monosaccharide esters, caramel, etc. The amounts of the monosaccharides, monosaccharide esters, and caramel in the SE can be determined by gas chromatography.

The ash content in the low-substitution-degree SE obtained by each process of the invention is generally 6.0% by weight or lower, preferably 5.0% by weight or lower, more preferably 2.0% by weight or lower. The lower limit of the ash content is generally 0.1% by weight. Ash means an ignition residue. Ash content means the amount of substances remaining after the ignition of 2-4 g of a sample mixed with sulfuric acid. For example, when sulfuric acid is added to 3 g of a sample and heating the mixture at 450-550°C for 3 hours results in a residue weight of 0.15 g, then the ash content is (0.15 g/3 g) $\times$100 = 5% by weight. Specifically, ash content can be determined by "Ignition Residue Test Method" described in *Shokuhin Tenkabutsu Kōtei-sho Kaisetsu-sho,* 7th edition (Hirokawa Shoten) B-p.59.

EXAMPLE 1

The invention will be explained below in more detail by reference to Examples. However, the invention should not be construed as being limited to the Examples unless the invention departs from the spirit thereof.
In the following Examples, each % is % by weight.

EXAMPLE 1

Preparation of Activated Sucrose.

Into an eggplant type flask were introduced 250.0 g of sucrose, 75.0 g of KOH, 305.3 g of palmitic acid, 790.0 g of methanol, and 290.0 g of water. The contents were mixed for 30 minutes with heating at 60°C to thereby dissolve the ingredients. (Here, almost all of the palmitic acid reacted with part of the KOH to yield the potassium soap of palmitic acid, and the remaining KOH was used for activating the sucrose.) The resultant liquid was dried under vacuum with an evaporator at 60°C so as to prevent the sucrose from decomposing and was thereby solidified. (When foaming occurred due to the palmitic acid soap during this vacuum drying, the methanol and water were removed while diminishing the foam by $N_2$ stripping.) The white powder obtained was pulverized with a mortar. The water content of the white powder was determined by Karl Fisher's method and, as a result, was found to be 0.1% by weight or lower.

Reaction of Raw SE with Activated Sucrose

Into a round-bottom eggplant type flask were introduced 26.9 g of the white powder containing an activated sucrose prepared by the method shown above, 5.0 g of a sucrose fatty acid ester ("RYOTO Sugar Ester S570" manufactured by Mitsubishi-Kagaku Foods Corp.; proportion of constituent fatty acids, stearic acid/palmitic acid = 70/30; average degree of substitution (found value), 2.22), and 21.3 g of methyl ethyl ketone (MEK) at ordinary temperature. While the atmosphere in this round-bottom eggplant type flask was kept being an $N_2$ atmosphere, a 90°C oil bath was used to heat the liquid reaction mixture in the flask with sufficient stirring at 410 rpm with a motor-operated stirring blade (semicircular shape). Under these conditions, the reaction mixture was reacted at ordinary pressure for 6.0 hours while wholly refluxing the MEK. With respect to the state of the liquid reaction mixture during the reaction, a slight viscosity increase was observed with the progress of the reaction. However, the operation was free from problems, e.g., the twining of the liquid reaction mixture around the semicircular stirring blade, and the reaction mixture retained the state of being capable of sufficient stirring.

At the time when the reaction was initiated and at each of 4.0 hours after and 6.0 hours after initiation of the reaction, part (3 g) of the liquid reaction mixture was taken out, cooled with ice to terminate the reaction, and analyzed by gel permeation chromatography (GPC) and gas chromatography (GC).

The GPC analysis was conducted by the following procedure. First, MEK was added to the liquid reaction mixture in an amount three times by weight the amount of the liquid reaction mixture and a 0.2% by weight aqueous solution of potassium lactate was further added. After the ingredients were dispersed, this mixture was heated for dissolution. Subsequently, the pH of the aqueous phase was adjusted to 4 or lower with lactic acid to convert the palmitic acid soap to the fatty acid (palmitic acid) and remove the salt and sucrose by extraction with the aqueous phase. Furthermore, the MEK phase was dried and solidified, and the dry solid (containing SE and fatty acid) was subjected to GPC analysis. The results thereof are shown in Table 1. These results were examined for the ester distribution of the SE.

[Table 1]

Table 1: Ester distribution (%) and average degree of substitution of SE in reaction mixture

| Reaction time (hr) | 0.0 | 4.0 | 6.0 |
|---|---|---|---|
| mono | 27.7 | 74.1 | 77.0 |
| di | 36.6 | 22.6 | 20.9 |
| tri | 24.1 | 3.0 | 2.1 |
| tetra | 9.4 | 0.3 | 0.0 |
| penta | 2.1 | 0.0 | 0.0 |
| Average degree of substitution | 2.22 | 1.29 | 1.25 |

The average degree of substitution was calculated using the following equation.

$$
\begin{aligned}
\text{Average degree of substitution} = [&(\text{amount of mono-substituted form (wt\%)}) \times 1 \\
&+ (\text{amount of di-substituted form (wt\%)}) \times 2 \\
&+ (\text{amount of tri-substituted form (wt\%)}) \times 3 \\
&+ (\text{amount of tetra-substituted form (wt\%)}) \times 4 \\
&+ (\text{amount of penta-substituted form (wt\%)}) \times 5] \times 0.01
\end{aligned}
$$

The GC analysis was conducted by the trimethylsilylation method. With respect to details of the trimethylsilylation method, see, for example, Shishitsu Bunseki Ho Nyumon, 4th edition, Japan Scientific Societies Press, pp.178-180, January 15, 1985. The amounts of sucrose and the fatty acid were determined from the results of this analysis. The results of the analysis of the reaction mixture by GC are shown in Table 2.

A sample for the GC analysis was prepared in the following manner. Fifty milligrams of the liquid reaction mixture was precisely weighed out, and 1 mL of a pyridine solution containing 5 mg/mL squalane dissolved therein was added as an internal reference thereto. The resultant mixture was heated to 70°C to dissolve the reaction mixture. Thereto were added 0.7 mL of 1,1,1,3,3,3-hexamethyldesilazane and 0.5 mL of trimethylchlorosilane in this order. The resultant mixture was stirred, heated at 70°C for 10 minutes, and then allowed to stand at room temperature. Two microliters of the resultant supernatant were subjected to GC analysis. The amount of palmitic acid was determined by GC, and the amount of the potassium salt thereof was taken as the amount of potassium palmitate.

[Table 2]

Table 2: Proportions of substances in reaction mixture (%)

| Reaction time (hr) | 0.0 | 6.0 |
|---|---|---|

(continued)

| SE | 9.5 | 10.7 |
|---|---|---|
| Sucrose | 21.0 | 19.8 |
| Potassium palmitate | 29.3 | 29.0 |
| MEK | 40.2 | 39.8 |

It was ascertained from the results given above that the reaction of the raw SE with the activated sucrose had proceeded and the degree of substitution of the SE had decreased to yield an SE having an HLB of 15. Namely, an SE having an HLB of 15 could be synthesized without especially encountering a problem concerning a viscosity increase in the liquid reaction mixture.

The SE yielded had almost the same color as the raw SE, and no coloring by the reaction mixture was observed. The SE contained MEK in an amount of 1.0 ppm. The SE had an ash content of 2.0% by weight.

EXAMPLE 2

Synthesis of Raw SE

An SE which had an average degree of substitution (found value) of 2.11 and in which the proportion of constituent fatty acids was palmitic acid/stearic acid = 82/18 was synthesized in the following manner.

Into a reaction vessel were introduced 7, 000 g of DMSO, 1,320 g of sucrose, 1,680 g of fatty acid methyl esters (proportion of constituent fatty acids: palmitic acid/stearic acid = 82/18), and 25 g of anhydrous potassium carbonate. The resultant mixture was reacted at a pressure of 2.67 kPa while refluxing the DMSO at 88-92°C until 99% or more of the fatty acid methyl esters reacted and disappeared. The mixture was thus reacted for 7.0 hours. After completion of the reaction, 50% by weight aqueous lactic acid solution was added thereto to adjust the pH to 6.0. This liquid was supplied to an evaporator to vaporize the DMSO at 90°C and a pressure of 1.73 kPa. The composition obtained was subjected to GC analysis. As a result, the composition was found to be composed of 30% by weight DMSO, 7% by weight unreacted sucrose, 62% by weight SE, and 1% by weight other ingredients. To this composition were added isobutanol and water (containing 2,000 ppm potassium lactate) each in an amount three times by weight the amount of the composition. An extraction treatment was conducted at 60°C and ordinary pressure to remove the unreacted sucrose by dissolving it in the aqueous phase side. Furthermore, the isobutanol phase (upper phase) was subjected to liquid/liquid extraction with water (containing 2,000 ppm potassium lactate) at 60°C and ordinary pressure to remove the DMSO. The isobutanol phase obtained through this extraction was supplied to a thin-film evaporator to remove the isobutanol at 13.3 kPa to 2% by weight. A 3-fold amount of water was added to the residue and the resultant mixture was evaporated and dehydrated. Thus, a sucrose fatty acid ester having an average degree of substitution (found value) of 2.11 (proportion of constituent fatty acids: palmitic acid/stearic acid = 82/18) was obtained.

Reaction of Raw SE with Activated Sucrose

Reaction and analyses were conducted under the same conditions as in Example 1, except that use was made of 5.0 g of the SE obtained by the method described above, 35.9 g of the white powder containing an activated sucrose obtained in Example 1, and 27.2 g of MEK. The reaction was terminated at 6.0 hours after initiation to obtain an SE having an average degree of substitution of 1.26. The results of the analyses are shown in the following Tables 3 and 4. The degree of substitution and the amount of potassium palmitate were calculated in the same manners as in Example 1.

[Table 3]

Table 3: Ester distribution (%) and average degree of substitution of SE in reaction mixture

| Reaction time (hr) | 0.0 | 4.0 | 6.0 |
|---|---|---|---|
| mono | 29.8 | 74.2 | 76.3 |
| di | 38.7 | 22.6 | 21.3 |
| tri | 22.9 | 2.9 | 2.2 |
| tetra | 7.5 | 0.3 | 0.2 |
| penta | 1.1 | 0.0 | 0.0 |

(continued)

| Average degree of substitution | 2.11 | 1.29 | 1.26 |
|---|---|---|---|

[Table 4]

Table 4: Proportions of substances in reaction mixture (%)

| Reaction time (hr) | 0.0 | 6.0 |
|---|---|---|
| SE | 7.3 | 12.4 |
| Sucrose | 21.6 | 17.1 |
| Potassium palmitate | 30.4 | 30.2 |
| MEK | 40.0 | 39.7 |

It was ascertained from the results given above that the reaction of the raw SE obtained by the direct process with the activated sucrose had proceeded and the degree of substitution of the SE had decreased to yield an SE having an HLB of 15. Namely, an SE having an HLB of 15 could be synthesized without especially encountering a problem concerning a viscosity increase in the liquid reaction mixture.

The SE yielded had almost the same color as the raw SE, and no coloring by the reaction mixture was observed. The SE contained MEK in an amount of 1.0 ppm. The SE had an ash content of 2.1% by weight.

EXAMPLE 3

Into a round-bottom eggplant type flask were introduced 35.9 g of the white powder containing an activated sucrose used in Examples 1 and 2, 5.0 g of the SE having an average degree of substitution of 2.11 used in Example 2, and 75.8 g of toluene at ordinary temperature. While the atmosphere in this round-bottom eggplant type flask was kept being an $N_2$ atmosphere, a 90°C oil bath was used to heat the liquid reaction mixture in the flask with sufficient stirring at 410 rpm with a motor-operated stirring blade (semicircular shape). Under these conditions, the reaction mixture was reacted at ordinary pressure for 9.0 hours while wholly refluxing the toluene. A slight viscosity increase in the liquid reaction mixture was observed with the progress of the reaction. However, the operation was free from problems, e.g., the twining of the liquid reaction mixture around the semicircular stirring blade, and the reaction mixture retained the state of being capable of sufficient stirring. At the time when the reaction was initiated and at each of 2.0 hours, 4.0 hours, 6.0 hours, and 9. 0 hours after initiation of the reaction, the liquid reaction mixture was analyzed by the same methods as in Example 1. The results of the examination are shown in Tables 5 and 6.

[Table 5]

Table 5: Ester distribution (%) and average degree of substitution of SE in reaction mixture

| Reaction time (hr) | 0.0 | 2.0 | 4.0 | 6.0 | 9.0 |
|---|---|---|---|---|---|
| mono | 29.8 | 61.5 | 78.2 | 83.3 | 84.1 |
| di | 38.7 | 28.0 | 18.7 | 15.2 | 14.6 |
| tri | 22.9 | 7.7 | 2.3 | 1.0 | 0.9 |
| tetra | 7.5 | 2.0 | 0.4 | 0.5 | 0.5 |
| penta | 1.1 | 0.6 | 0.4 | 0.0 | 0.0 |
| Average degree of substitution | 2.11 | 1.52 | 1.26 | 1.19 | 1.18 |

[Table 6]

Table 6: Proportions of substances in reaction mixture (%)

| Reaction time (hr) | 0.0 | 9.0 |
|---|---|---|
| SE | 4.3 | 5.2 |
| Sucrose | 12.6 | 11.4 |

(continued)

| Potassium palmitate | 17.7 | 17.8 |
| Toluene | 65.0 | 65.2 |

It was ascertained from the results given above that the reaction of the SE with the activated sucrose had proceeded due to the use of the solvent having a low dielectric constant, and the degree of substitution of the SE had decreased to yield an SE having an HLB of 16. Namely, a low-substitution-degree SE having an HLB of 16 could be synthesized without especially encountering a problem concerning a viscosity increase in the liquid reaction mixture.

The SE yielded had almost the same color as the raw SE, and no coloring by the reaction mixture was observed. The SE contained toluene in an amount of 1.0 ppm. The SE had an ash content of 2.3% by weight.

EXAMPLE 4

An SE having an average degree of substitution of 1.43 obtained by the direct process was used as a raw material to conduct a reaction under the same conditions as in Example 1, and analyses were conducted under the same conditions as in Example 1. The activated sucrose which remained unreacted after the reaction was removed by water extraction, and the MEK was removed by distillation. As a result, an SE having an average degree of substitution (found value) of 1.21 was obtained. The low-substitution-degree SE obtained contained MEK in an amount of 1.5 ppm. No solvent other than the solvent used in the reaction in the process of the invention (provided that water, MeOH, and EtOH are excluded) was detected from the SE. The SE had an ash content of 2.5% by weight.

EXAMPLE 5

Into a round-bottom eggplant type flask were introduced 5.0 g of the SE having an average degree of substitution of 2.11 used in Example 2, 15.1 g of an activated sucrose (one obtained by dissolving 100 g of sucrose and 2.35 g of KOH having a purity of 86% by weight in a mixed solvent composed of 100 g of water and 100 g of ethanol, drying/solidifying the solution under vacuum at 90°C, and pulverizing the resultant solid with a mortar), 27.2 g of MEK, and 20.7 g of stearic acid monoglyceride ("Emasol MP-V" manufactured by Riken Vitamin Co., Ltd.) at ordinary temperature. While the atmosphere in this round-bottom eggplant type flask was kept being an $N_2$ atmosphere, a 90°C oil bath was used to heat the liquid reaction mixture in the flask with sufficient stirring at 200 rpm with a motor-operated stirring blade (semicircular shape). Under these conditions, the reaction mixture was reacted at ordinary pressure for 6.0 hours while wholly refluxing the MEK. With respect to the state of the liquid reaction mixture during the reaction, the operation was free from problems, e.g., the twining of the liquid reaction mixture around the semicircular stirring blade, and the reaction mixture retained the state of being capable of sufficient stirring. At 6.0 hours after reaction initiation, the liquid reaction mixture was analyzed by the same methods as in Example 1. The SE which had been reacted for 6.0 hours had an average degree of substitution of 1.23. This SE had an ash content of 0.8% by weight.

COMPARATIVE EXAMPLE 1

Into a round-bottom eggplant type flask were introduced 12.4 g of sucrose (highly refined sugar) pulverized with a mortar, 13.1 g of the SE having a degree of substitution of 2.22 used in Example 1, 10.0 g of MEK, 4.5 g of potassium stearate, and 0.43 g of potassium carbonate ($K_2CO_3$) at ordinary temperature. While the atmosphere in this round-bottom eggplant type flask was kept being an $N_2$ atmosphere, a 90°C oil bath was used to heat the liquid reaction mixture in the flask with sufficient stirring at 410 rpm with a motor-operated stirring blade (semicircular shape). Under these conditions, the reaction mixture was reacted at ordinary pressure for 8.0 hours while wholly refluxing the toluene. At the time when the reaction was initiated and at 8.0 hours after initiation of the reaction, the liquid reaction mixture was analyzed by the same methods as in Example 1. The results of the examination are shown in Table 7.

[Table 7]

Table 7: Ester distribution (%) and average degree of substitution of SE in reaction mixture

| Reaction time (hr) | 0.0 | 8.0 |
|---|---|---|
| mono | 27.7 | 23.0 |
| di | 36.6 | 38.4 |
| tri | 24.1 | 26.3 |

(continued)

| | | |
|---|---|---|
| tetra | 9.4 | 9.75 |
| penta | 2.1 | 2.2 |
| hexa | 0.0 | 0.34 |
| Average degree of substitution | 2.22 | 2.31 |

The results given in Table 7 show that the degree of substitution of the SE had been slightly heightened by the reaction. It was hence ascertained that when an SE is to be reacted with sucrose in the presence of the solvent for use in a process of the invention, the sucrose should be an activated sucrose.

COMPARATIVE EXAMPLE 2

Into a round-bottom eggplant type flask were introduced 12.4 g of sucrose (highly refined sugar) pulverized with a mortar, 13.1 g of the SE having a degree of substitution of 2.22 used in Example 1, 18.5 g of MEK, 17.4 g of potassium palmitate, and 0.42 g of potassium methoxide at ordinary temperature. While the atmosphere in this round-bottom eggplant type flask was kept being an $N_2$ atmosphere, a 90°C oil bath was used to heat the liquid reaction mixture in the flask with sufficient stirring at 410 rpm with a motor-operated stirring blade (semicircular shape). Under these conditions, the reaction mixture was reacted at ordinary pressure for 6.0 hours while wholly refluxing the toluene. At the time when the reaction was initiated and at 4.0 hours after initiation of the reaction, the liquid reaction mixture was analyzed by the same methods as in Example 1. The results of the examination are shown in Table 8.

GC analysis was further conducted. As a result, the formation of an extremely slight amount of a fatty acid methyl ester and an extremely slight decrease in the degree of substitution of the SE were observed. However, the sucrose and the SE had undergone no reaction between these such as those shown in Examples 1 to 4. It was ascertained also from these results that when an SE is to be reacted with sucrose in the presence of the solvent for use in a process of the invention, the sucrose should be an activated sucrose.

[Table 8]

Table 8: Ester distribution (%) and average degree of substitution of SE in reaction mixture

| Reaction time (hr) | 0.0 | 4.0 |
|---|---|---|
| mono | 27.7 | 29.7 |
| di | 36.6 | 38.0 |
| tri | 24.1 | 22.5 |
| tetra | 9.4 | 7.9 |
| penta | 2.1 | 1.6 |
| hexa | 0.0 | 0.3 |
| Average degree of substitution | 2.22 | 2.14 |

REFERENCE EXAMPLE

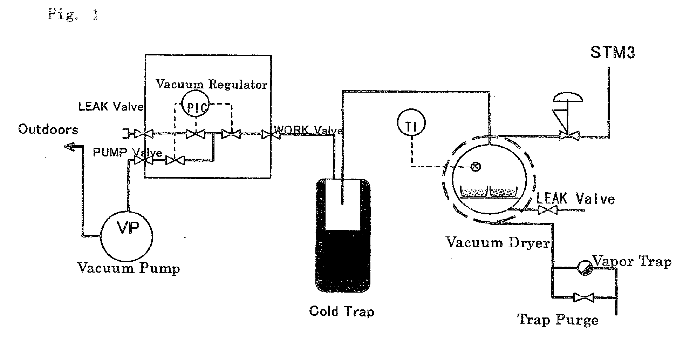

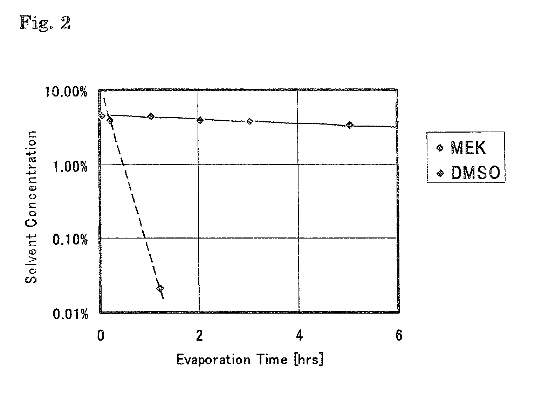

The evaporation rates of MEK and DMSO were compared. Specifically, the procedure is as follows. In a sample bottle were placed 100 g of a sucrose fatty acid ester ("RYOTO Sugar Ester P1670" manufactured by Mitsubishi-Kagaku Foods Corp.; proportion of constituent fatty acids, palmitic acid/stearic acid = 82/18; average degree of substitution (found value), 1.21) and 5 g of MEK or DMSO. This sample bottle in a closed state was stored for 7 days. Five grams of this SE containing DMSO or MEK was put in a 58-mm$\phi$ cylindrical aluminum cup so as to evenly and flatly spread on the bottom, and was weighed. Six such aluminum cups were prepared and placed in a vacuum dryer kept at 90°C (the apparatus is diagrammatically shown in Fig. 1). This vacuum dryer was evacuated to 50 Torr (6.7 kPa). After initiation of the evacuation, the samples were taken out one by one at intervals of 1 hour. Each sample was weighed, subsequently ground with a mortar, and subjected to GC analysis to determine the amount of the residual solvent. The results thereof are shown in Fig. 2. The results show that MEK is far more apt to evaporate than DMSO. It was thus found that the removal of the solvent for use in a process of the invention is easy.

While the invention has been described in detail and with reference to specific embodiments thereof, it will be apparent to one skilled in the art that various changes and modifications can be made therein without departing from the spirit and scope thereof.

This application is based on a Japanese patent application filed on November 19, 2004 (Application No. 2004-336310), the contents thereof being herein incorporated by reference.

INDUSTRIAL APPLICABILITY

The invention provides processes which are useful for obtaining a sucrose fatty acid ester having high quality and a low degree of substitution and suitable for use as, e.g., an emulsifying agent for foods and which eliminate the necessity of the complicated solvent removal operation in related-art processes and are industrially superior.

**Claims**

1. A production process of a sucrose fatty acid ester having a low degree of substitution, wherein the production process comprises reacting the raw sucrose fatty acid ester with an activated sucrose in the presence of a solvent having a dielectric constant at 20°C of 35.0 or lower and having no hydroxyl group,
whereby the average degree of substitution of the raw sucrose fatty acid ester is lowered by 0.2 or more, and wherein the sucrose fatty acid ester having a low degree of substitution to be yielded has an average degree of substitution of 1.0 to 2.0.

2. The production process of claim 1, which comprises using a solvent having a dielectric constant at 20°C of 1.1 to 20.0.

3. A process for producing a sucrose fatty acid ester having a low degree of substitution, wherein the production process comprises reacting the raw sucrose fatty acid ester with an activated sucrose in the presence of a solvent having a boiling point at ordinary pressure of 150°C or lower and a solubility in water at 20°C of 50 g/100 mL or lower and having no hydroxyl group,
whereby the average degree of substitution of the raw sucrose fatty acid ester is lowered by 0.2 or more, and wherein the sucrose fatty acid ester having a low degree of substitution to be yielded has an average degree of substitution of 1.0 to 2.0.

4. The production process of claim 1, wherein the sucrose fatty acid ester having a low degree of substitution to be yielded has an average degree of substitution of 1.0 to 1.6.

5. The production process of claim 3, wherein the sucrose fatty acid ester having a low degree of substitution to be yielded has an average degree of substitution of 1.0 to 1.6.

6. The production process of claim 1, wherein the raw sucrose fatty acid ester has an average degree of substitution of 1.5 to 5.0.

7. The production process of claim 3, wherein the raw sucrose fatty acid ester has an average degree of substitution of 1.5 to 5.0.

8. The production process of claim 1, wherein the raw sucrose fatty acid ester is an ester of a fatty acid having 8 to 22 carbon atoms.

9. The production process of claim 3, wherein the raw sucrose fatty acid ester is an ester of a fatty acid having 8 to 22 carbon atoms.

10. The production process of claim 1, wherein the raw sucrose fatty acid ester is an ester of palmitic acid.

11. The production process of claim 3, wherein the raw sucrose fatty acid ester is an ester of palmitic acid.

12. The production process of claim 1, wherein the reaction of the raw sucrose fatty acid ester with the activated sucrose is conducted in the presence of an emulsifying agent.

13. The production process of claim 3, wherein the reaction of the raw sucrose fatty acid ester with the activated sucrose is conducted in the presence of an emulsifying agent.

**Patentansprüche**

1. Ein Verfahren zur Herstellung eines Saccharose-Fettsäureesters mit einem geringen Substitutionsgrad, wobei das Herstellungsverfahren Umsetzen des rohen Saccharose-Fettsäureesters mit einer aktivierten Saccharose in Gegenwart eines Lösungsmittels mit einer Dielektrizitätskonstante bei 20°C von 35,0 oder weniger und ohne Hydroxylgruppen umfasst, wobei der mittlere Substitutionsgrad des rohen Saccharose-Fettsäureesters um 0,2 oder mehr verringert wird, und wobei der zu gewinnende Saccharose-Fettsäureester mit einem geringen Substitutionsgrad einen mittleren Substitutionsgrad von 1,0 bis 2,0 aufweist.

2. Das Herstellungsverfahren nach Anspruch 1, welches die Verwendung eines Lösungsmittels mit einer Dielektrizitätskonstante bei 20°C von 1,1 bis 20,0 umfasst.

3. Ein Verfahren zur Herstellung eines Saccharose-Fettsäureesters mit einem geringen Substitutionsgrad, wobei das Herstellungsverfahren Umsetzen des rohen Saccharose-Fettsäureesters mit einer aktivierten Saccharose in Gegenwart eines Lösungsmittels mit einem Siedepunkt bei gewöhnlichem Druck von 150°C oder weniger und einer Löslichkeit in Wasser bei 20°C von 50 g/100 ml oder weniger und ohne Hydroxylgruppen umfasst, wobei der mittlere Substitutionsgrad des rohen Saccharose-Fettsäureesters um 0,2 oder mehr verringert wird, und wobei der zu gewinnende Saccharose-Fettsäureester mit einem geringen Substitutionsgrad einen mittleren Substitutionsgrad von 1,0 bis 2,0 aufweist.

4. Das Herstellungsverfahren nach Anspruch 1, wobei der zu gewinnende Saccharose-Fettsäureester mit einem geringen Substitutionsgrad einen mittleren Substitutionsgrad von 1,0 bis 1,6 aufweist.

5. Das Herstellungsverfahren nach Anspruch 3, wobei der zu gewinnende Saccharose-Fettsäureester mit einem geringen Substitutionsgrad einen mittleren Substitutionsgrad von 1,0 bis 1,6 aufweist.

6. Das Herstellungsverfahren nach Anspruch 1, wobei der rohe Saccharose-Fettsäureester einen mittleren Substitutionsgrad von 1,5 bis 5,0 aufweist.

7. Das Herstellungsverfahren nach Anspruch 3, wobei der rohe Saccharose-Fettsäureester einen mittleren Substitutionsgrad von 1,5 bis 5,0 aufweist.

8. Das Herstellungsverfahren nach Anspruch 1, wobei der rohe Saccharose-Fettsäureester ein Ester einer Fettsäure mit 8 bis 22 Kohlenstoffatomen ist.

9. Das Herstellungsverfahren nach Anspruch 3, wobei der rohe Saccharose-Fettsäureester ein Ester einer Fettsäure mit 8 bis 22 Kohlenstoffatomen ist.

10. Das Herstellungsverfahren nach Anspruch 1, wobei der rohe Saccharose-Fettsäureester ein Ester von Palmitinsäure ist.

11. Das Herstellungsverfahren nach Anspruch 3, wobei der rohe Saccharose-Fettsäureester ein Ester von Palmitinsäure ist.

12. Das Herstellungsverfahren nach Anspruch 1, wobei das Umsetzen des rohen Saccharose-Fettsäureesters mit der aktivierten Saccharose in Gegenwart eines Emulgators erfolgt.

13. Das Herstellungsverfahren nach Anspruch 3, wobei das Umsetzen des rohen Saccharose-Fettsäureesters mit der aktivierten Saccharose in Gegenwart eines Emulgators erfolgt.

**Revendications**

1. Procédé de production d'un ester d'acide gras et de saccharose ayant un faible degré de substitution, dans lequel le procédé de production comprend la mise en réaction de l'ester d'acide gras et de saccharose brut avec un saccharose activé en présence d'un solvant ayant une constante diélectrique à 20 °C inférieure ou égale à 35,0 et n'ayant pas de groupe hydroxyle,

par lequel le degré moyen de substitution de l'ester d'acide gras et de saccharose brut est diminué de 0,2 ou plus, et dans lequel l'ester d'acide gras et de saccharose devant atteindre un faible degré de substitution, a un degré moyen de substitution de 1,0 à 2,0.

2.  Procédé de production selon la revendication 1, qui comprend l'utilisation d'un solvant ayant une constante diélectrique à 20 °C de 1,1 à 20,0.

3.  Procédé de production d'un ester d'acide gras et de saccharose ayant un faible degré de substitution, dans lequel le procédé de production comprend la mise en réaction de l'ester d'acide gras et de saccharose brut avec un saccharose activé en présence d'un solvant ayant un point d'ébullition à pression normale de 150 °C ou moins et une solubilité dans l'eau à 20 °C de 50 g/100 mL ou moins et n'ayant pas de groupe hydroxyle,
    par lequel le degré moyen de substitution de l'ester d'acide gras et de saccharose brut est diminué de 0,2 ou plus, et dans lequel l'ester d'acide gras et de saccharose devant atteindre un faible degré de substitution, a un degré moyen de substitution de 1,0 à 2,0.

4.  Procédé de production selon la revendication 1, dans lequel l'ester d'acide gras et de saccharose devant atteindre un faible degré de substitution, a un degré moyen de substitution de 1,0 à 1,6.

5.  Procédé de production selon la revendication 3, dans lequel l'ester d'acide gras et de saccharose devant atteindre un faible degré de substitution, a un degré moyen de substitution de 1,0 à 1,6.

6.  Procédé de production selon la revendication 1, dans lequel l'ester d'acide gras et de saccharose brut a un degré moyen de substitution de 1,5 à 5,0.

7.  Procédé de production selon la revendication 3, dans lequel l'ester d'acide gras et de saccharose brut a un degré moyen de substitution de 1,5 à 5,0.

8.  Procédé de production selon la revendication 1, dans lequel l'ester d'acide gras et de saccharose brut est un ester d'un acide gras ayant de 8 à 22 atomes de carbone.

9.  Procédé de production selon la revendication 3, dans lequel l'ester d'acide gras et de saccharose brut est un ester d'un acide gras ayant de 8 à 22 atomes de carbone.

10. Procédé de production selon la revendication 1, dans lequel l'ester d'acide gras et de saccharose brut est un ester de l'acide palmitique.

11. Procédé de production selon la revendication 3, dans lequel l'ester d'acide gras et de saccharose brut est un ester de l'acide palmitique.

12. Procédé de production selon la revendication 1, dans lequel la réaction de l'ester d'acide gras et de saccharose brut avec le saccharose activé est réalisée en présence d'un agent émulsifiant.

13. Procédé de production selon la revendication 3, dans lequel la réaction de l'ester d'acide gras et de saccharose brut avec le saccharose activé est réalisée en présence d'un agent émulsifiant.

Fig. 1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 51014485 B
- JP 4065080 B
- JP 59078200 A
- EP 0560081 A
- JP 2004336310 A

### Non-patent literature cited in the description

- Kagaku Binran. 619-623
- Kagaku Binran. 498-501
- **ARNI et al.** *J. Appl. Chem.,* 09 March 1959, 186-192
- **J.A. RENDLEMAN, JR.** *Adv. In Carbohydr. Chem.,* 1966, vol. 21, 209-269
- Shishitsu Bunseki Ho Nyumon. Japan Scientific Societies Press, 15 January 1985, 178-180